(19)

Europäisches
Patentamt

European
Patent Office

Office européen
des brevets

(11)  **EP 2 197 865 B1**

(12)  **EUROPEAN PATENT SPECIFICATION**

(45) Date of publication and mention
of the grant of the patent:
**22.08.2012 Bulletin 2012/34**

(21) Application number: **08801979.9**

(22) Date of filing: **05.09.2008**

(51) Int Cl.:
*C07D 307/46* (2006.01)   *C10L 1/02* (2006.01)

(86) International application number:
**PCT/EP2008/007411**

(87) International publication number:
**WO 2009/030505 (12.03.2009 Gazette 2009/11)**

(54) **HYDROXYMETHYLFURFURAL ETHERS FROM HMF AND OLEFINS**

HYDROXYMETHYLFURFURALETHER AUS HMF UND OLEFINEN

ETHERS HYDROXYMÉTHYLFURFURAUX DES HMF ET OLÉFINES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **07.09.2007 EP 07075772**

(43) Date of publication of application:
**23.06.2010 Bulletin 2010/25**

(73) Proprietor: **Furanix Technologies B.V
1014 BV Amsterdam (NL)**

(72) Inventor: **GRUTER, Gerardus, Johannes, Maria
NL-2106 BA Heemstede (NL)**

(74) Representative: **Kortekaas, Marcel C.J.A.
Exter Polak & Charlouis B.V. (EP&C)
P.O. Box 3241
2280 GE Rijswijk (NL)**

(56) References cited:
**EP-A- 1 834 950          WO-A-99/67409
WO-A-2006/063220    WO-A-2007/104514
DE-A1- 3 621 517**

- **GARVES K: "Acid catalyzed degradation of
cellulose in alcohols" JOURNAL OF WOOD
CHEMISTRY AND TECHNOLOGY, MARCEL
DEKKER, NEW YORK, NY, US, vol. 8, no. 1, 1988,
pages 121-134, XP009067962 ISSN: 0277-3813**
- **SADAO TSUBOI ET AL: BULLETIN OF THE
CHEMICAL SOCIETY OF JAPAN, vol. 60, 1987,
pages 1807-1812, XP002468609**
- **FILHO J R DE FREITAS ET AL: "Synthesis of new
2,3-unsaturated o-glycosides through ferrier
rearrangement" JOURNAL OF CARBOHYDRATE
CHEMISTRY, NEW YORK, NY, US, vol. 20, no. 7-8,
2001, pages 561-568, XP009095954 ISSN:
0732-8303**
- **DRECHSLER G ET AL: "Über die Reaktion von
Furfurolderivaten mit Acrylnitril in alkalischem
Medium" JOURNAL FUER PRAKTISCHE
CHEMIE, WILEY VCH, WEINHEIM, vol. 27, 1
January 1965 (1965-01-01), pages 258-270,
XP002487794 ISSN: 1436-9966**

## Description

<u>Technical Field</u>

The present invention concerns a method for the manufacture of an ether of 5-hydroxymethylfurfural (5-(hydroxymethyl)-2-furaldehyde, or HMF) from HMF and olefins.

<u>Background Art</u>

Fuel, fuel additives and various chemicals used in the petrochemical industry are derived from oil, gas and coal, all finite sources. Biomass, on the other hand, is considered a renewable source. Biomass is biological material (including biodegradable wastes) which can be used for the production of fuels or for industrial production of e.g. fibres, chemicals or heat. It excludes organic material which has been transformed by geological processes into substances such as coal or petroleum.

Production of biomass derived products for non-food applications is a growing industry as interest in sustainable fuel sources is growing. Bio based fuels are an example of an application with strong growing interest. Biomass contains sugars (hexoses and pentoses) that may be converted into value added products. Current biofuel activities from sugars are mainly directed towards the fermentation of sucrose or glucose into ethanol or via complete breakdown via Syngas to synthetic liquid fuels.

EP 0641 854 describes the use of fuel compositions comprising of hydrocarbons and/or vegetable oil derivatives containing at least one glycerol ether to reduce particulate matter emissions.

More recently, the acid catalysed reaction of fructose has been re-visited, creating HMF as an intermediate of great interest. Most processes investigated have the disadvantage that HMF is not very stable at the reaction conditions required for its formation. Fast removal from the water-phase containing the sugar starting material and the acid catalyst has been viewed as a solution for this problem. Researchers at the University of Wisconsin-Madison have developed a process to make HMF from fructose. HMF can be converted into monomers for plastics, petroleum or fuel extenders, or even into fuel itself. The process by prof. James Dumesic and co-workers first dehydrates the fructose in an aqueous phase with the use of an acid catalyst (hydrochloric acid or an acidic ion-exchange resin). Salt is added to salt-out the HMF into the extracting phase. The extracting phase uses an inert organic solvent that favors extraction of HMF from the aqueous phase. The two-phase process operates at high fructose concentrations (10 to 50 wt %), achieves high yields (80% HMF selectivity at 90% fructose conversion), and delivers HMF in a separation-friendly solvent ( DUMESIC, James A, et al. "Phase modifiers promote efficient production of Hydroxymethylfurfural from fructose" . Science. 30 juni 2006, vol.312, no.5782, p.1933-1937). Although the HMF yields from this process are interesting, the multi-solvent process has cost-disadvantages due to the relatively complex plant design and because of the less than ideal yields when cheaper and less reactive hexoses than fructose, such as glucose or sucrose, are used as a starting material. HMF is a solid at room temperature which has to be converted in subsequent steps to useful products. Dumesic has reported an integrated hydrogenolysis process step to convert HMF into dimethylfuran (DMF), which is assumed to be an interesting gasoline additive.

In WO 2006/063220 a method is provided for converting fructose into 5-ethoxymethylfurfural (EMF) at 60 °C, using an acid catalyst either in batch during 24 hours or continuously via column elution during 17 hours. Applications of EMF were not discussed.

Nonetheless, there remains an interest in a much more efficient and faster method for the manufacture of HMF ethers without the formation of by-products and uncontrolled degradation and without the restriction of using the ethanol reagent as the reaction solvent. The inventors have set out to overcome this shortfall.

In a companion patent application, the inventors have described a method for the manufacture of an ether of 5-hydroxymethylfurfural by reacting a fructose and/or glucosecontaining starting material with an olefin in the presence of an acid catalyst. This reaction is believed to proceed through the intermediate HMF, which is then reacted with olefin in the presence of the acid catalyst. The inventors have found that the same products may be made from HMF itself, for instance when the HMF is made by any of the prior art processes and isolated for further derivatization.

<u>Disclosure of Invention</u>

Accordingly, the current invention provides a method for the manufacture of an ether of 5-hydroxymethylfurfural through hydroxy-alkoxy-addition by reacting 5-hydroxymethylfurfural with an olefin in the presence of an acid catalyst, as defined in claim 1.

When the reaction product of the above method is used as such or when it is used as an intermediate for a subsequent conversion the selectivity of the reaction is preferably high as the product is preferably pure. However, when the reaction product of the above method is used as a fuel, a fuel additive or as a fuel or a fuel additive intermediate, the reaction product does not necessarily need to be pure. Indeed, the HMF starting material may contain non-interfering components or components with an alcoholic functionality which may be converted to their ethers at the same time. Other hydroxymethylfuran derivatives will also be converted into their corresponding tert-butoxymethyl ether derivatives. The ultimate reaction product may therefore contain non-interfering components such as other furan ethers and levulinic acid derivatives. For ease of reference, however, the method and the reaction product are described in terms of the reaction of HMF starting material with an olefin, resulting in an ether of HMF.

The reaction product made according to the present invention may be used as fuel or as fuel additive. Fuels for blending with the product of the present invention include but are not limited to gasoline and gasoline-ethanol blends, kerosene, diesel, biodiesel (refers to a non-petroleum-based diesel fuel consisting of short chain alkyl (methyl or ethyl) esters, made by transesterification of vegetable oil, which can be used (alone, or blended with conventional petrodiesel) in unmodified diesel-engine vehicles), Fischer-Tropsch liquids (for example obtained from GTL, CTL or BTL gas-to-liquids/coal-to-liquids/biomass to liquids processes), diesel-biodiesel blends and green diesel and blends of diesel and/or biodiesel with green diesel (green diesel is a hydrocarbon obtained by hydrotreating biomass derived oils, fats, greases or pyrolysis oil; see for example the UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085)). The product is a premium diesel fuel containing no sulfur and having a cetane number of 90 to 100). Fuels for blending with the product of the present invention may also include one or more other furanics, wherein the expression furanics is used to include all derivatives of furan and tetrahydrofuran. The HMF ethers made according to the invention can also be used as or can be converted to compounds that can be used as solvent, as monomer in a polymerization, as fine chemical intermediate, or in other applications.

Figure

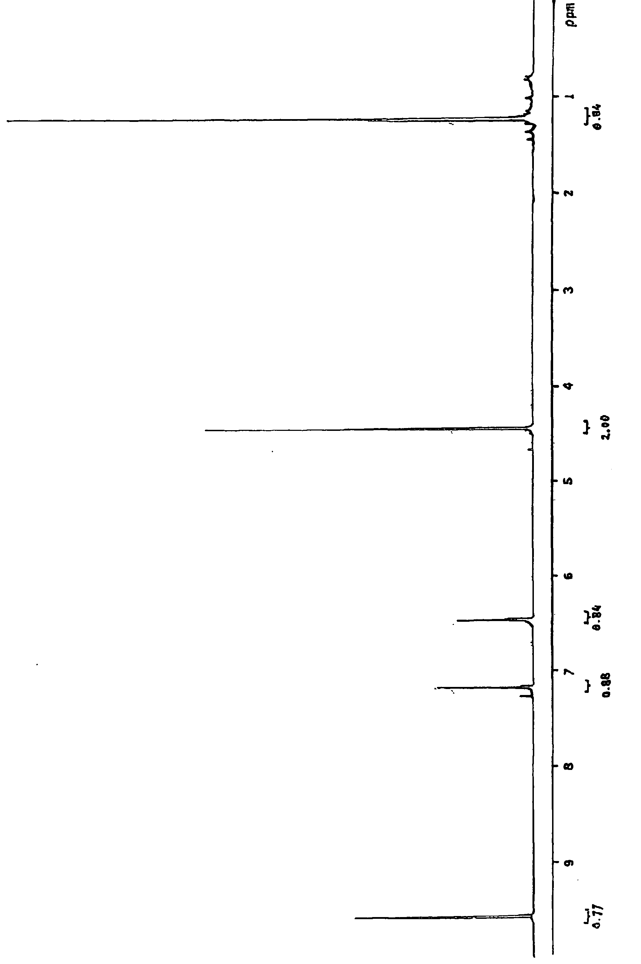

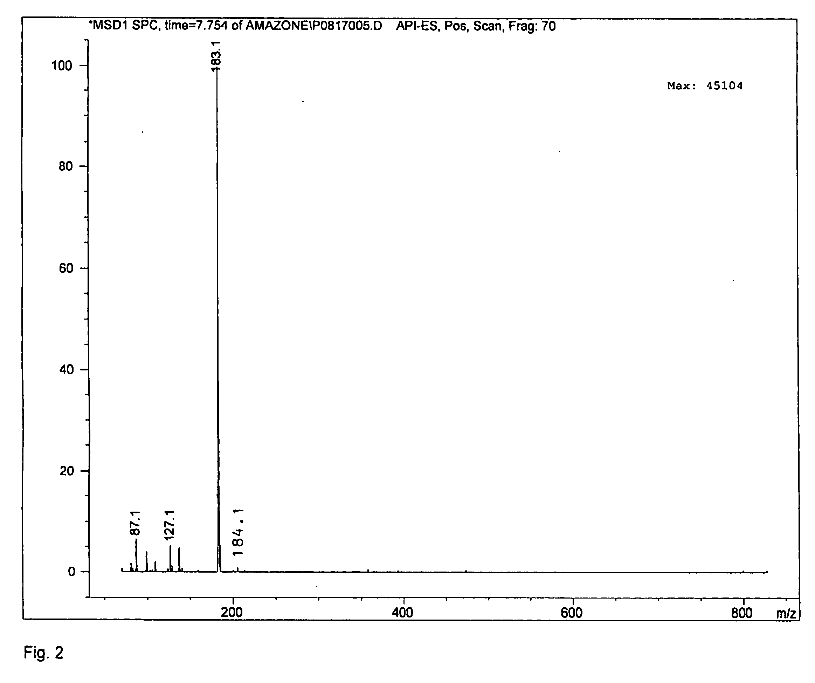

Figure 1 is the 1 H-NMR of 5-(tert-butoxymethyl)furfural, tBMF, prepared by the process of the current invention. Figure 2 is the spectrum of tBMF using a mass spectrometer in Chemical Ionization (C.I.) mode.

Mode(s) for Carrying Out the Invention

The synthesis of HMF from fructose, glucose and sucrose as a biomass source is a hot topic. HMF has been obtained in processes using both homogeneous and heterogeneous catalysts, using different diluent systems such as water, 2 phase systems for extracting the HMF into an organic phase after its formation, or using diluent systems such as acetone, dmso or ionic liquids.

The olefin used in the method of the current invention is preferably an olefinically unsaturated compound that is susceptible to electrophilic attack. It would thus appear that concurrent with the dehydration of the monosaccharide, a hydro-alkoxy-addition occurs. The addition of alcohols and phenols to the double bond of an olefinically unsaturated compound is discussed in Chapter 15 of Advanced Organic Chemistry, by Jerry March, and in particular under reaction 5-4. (3rd ed., © 1985 by John Wiley & Sons, pp. 684-685). This reference book, however, provides no information on the preparation of renewable liquid fuel (additives). Surprisingly, the in-situ preparation of the HMF is not hampered by the hydro-alkoxy-addition.

This reaction is therefore different, and the products are therefore different from the 2,3-unsaturated o-glycosides synthesized by Filho J.R. DE FREITAS et al, "Synthesis of new 2,3-unsaturated o-glycosides through ferrier rearrangement", in Journal of Carbohydrate Chemistry, 2001, vol. 20, pages 561-568. In this synthesis, an unsaturated o-glycal is converted into an unsaturated glycoside, by $S_n2'$ attack of the alcohol oxygen at C1, with subsequent elimination of the complexed O-acetyl group, as opposed to the hydro-alkoxy-addition where the unsaturated carbon-carbon bond is reacted away.

The olefins contain 4 carbon atoms or more. Isobutylene has been found to be very useful. Indeed, preferred olefins are cycloolefins and substituted iso olefins such as isobutene, 2-methyl-2-butene, 2-methyl-1-butene, 2-methyl-1-pentene, 2-methyl-2-pentene, 3-methyl-2-pentene, 2-ethyl-1-butene, 2,3-dimethyl-1-butene, 2,3-dimethyl-2-butene, 1-methylcyclopentene, the C7 iso olefins and similar C8 and higher olefins. Also suitable are dienes such as butadiene and isoprene and terpenes such as pinene and limonene. The olefins are represented by the general formulae

$$HRC=CR'R'' \quad H2C=CR'R''$$

wherein each R, R' and R'' independently represents an alkyl, aralkyl and alkenyl group which may be linear, branched or cyclic with up to 7 carbon atoms, or R and R' jointly represent an alkenyl group with up to 7 carbon atoms. Of these, substituted olefins having up to 8 carbon atoms in total are preferred, with isobutylene being most preferred. The iso olefins used as reagents are generally used in mixture with other hydrocarbons of similar boiling points. For instance isobutene feedstocks usually are C4 cuts from Fluid Catalytic Cracking (FCC) plants, from Steam Cracking plants or from field butanes Dehydrogenation/Isomerization plants. Depending on their origin, these C4 cuts usually contain between 20 and 50 wt% isobutene. The hydro-alkoxy addition reaction is highly selective so that nearly only the isoolefins are converted to ethers.

Another class of olefins found to be suitable are alkyl-substituted derivatives of cyclic olefins. These olefinically unsaturated compounds may contain elements other than carbon, such as oxygen, provided the olefinically unsaturated bond remains susceptible to electrophilic attack and provided that the other functional groups are compatible with the acid catalysed dehydration reactions and with the hydrolytic cleavage reactions.

The amount of olefin used during the manufacture of the HMF ether is preferably at least equimolar to the HMF content in the feedstock, but it is typically used in much greater excess. Indeed, the olefin may be used as solvent or co-solvent. In such a case, a sufficient amount of olefin is present to form the HMF ether. The catalyst is preferably selected such that the olefins are not reacting to dimers, oligomers and or polymers.

The acid catalyst in the method of the present invention can be selected from amongst (halogenated) organic acids, inorganic acids, Lewis acids, ion exchange resins and zeolites or combinations and/or mixtures thereof. It may be a homogeneous catalyst, but heterogeneous catalysts (meaning solid) are preferred for purification reasons. The HMF ethers can be produced with a protonic, Brønsted or, alternatively, a Lewis acid or with catalysts that have more than one of these acidic functionalities.

The protonic acid may be organic or inorganic. For instance, the organic acid can be selected from amongst oxalic acid, levulinic acid, maleic acid, trifluoro acetic acid (triflic acid), methanesulphonic acid or para-toluenesulphonic acid. Alternatively, the inorganic acid can be selected from amongst (poly)phosphoric acid, sulphuric acid, hydrochloric acid, hydrobromic acid, nitric acid, hydroiodic acid, optionally generated in situ.

Certain salts may be used as catalyst, wherein the salt can be any one or more of $(NH_4)_2SO_4/SO_3$, ammonium phosphate, pyridinium chloride, triethylamine phosphate, pyridinium salts, pyridinium phosphate, pyridinium hydrochloride/hydrobromide/perbromate, DMAP, aluminium salts, Th and Zr ions, zirconium phosphate, Sc and lanthanide ions such as Sm and Y as their acetate or trifluoroactate (triflate) salt, Cr-, Al-, Ti-, Ca-, In-ions, $ZrOCl_2$, $VO(SO_4)_2$, $TiO_2$, V-porphyrine, Zr-, Cr-, Ti-porphyrine.

Lewis acids selected as dehydration catalyst can be any one of $ZnCl_2$, $AlCl_3$, $BF_3$.

Ion exchange resins can be suitable dehydration catalysts. Examples include Amberlite™ and Amberlyst™, Diaion™ and Levatit™. Other solid catalyst that may be used include natural clay minerals, zeolites, supported acids such as silica impregnated with mineral acids, heat treated charcoal, metal oxides, metal sulfides, metal salts and mixed oxides and mixtures thereof. If elevated reactions temperatures are used, as defined hereafter, then the catalyst should be stable at these temperatures.

An overview of catalysts that may be used in the method of the current invention may be found in Table 1 of the review article prepared by Mr. Lewkowski: "Synthesis, chemistry and applications of 5-hydroxymethylfurfural and its derivatives" Arkivoc. 2001, p.17-54.

The amount of catalyst may vary, depending on the selection of catalyst or catalyst mixture. For instance, the catalyst can be added to the reaction mixture in an amount varying from 0.01 to 40 mole % drawn on the HMF content of the feedstock, preferably from 0.1 to 30 mole %, more preferably from 1 to 20 mole %.

In the preferred embodiment, the catalyst is a heterogeneous catalyst.

The temperature at which the reaction is performed may vary, but in general it is preferred that the reaction is carried out at a temperature from 50 to 200 degrees Celsius, preferably from 50 to 150 degrees Celsius, more preferably from 75 to 125 degrees Celsius. In general, temperatures higher than 150 degrees are less preferred as the selectivity of the reaction reduces and as many by-products occur. Performing the reaction below the lowest temperature is also less preferable because of the low reaction rate. If the reactions are carried out above the boiling temperature of water, then the reactions are preferably carried out under pressure, e.g., 10 bar nitrogen or higher.

The HMF starting material is typically dissolved in a solvent, which can also be the olefin reactant, in order to facilitate the reaction. The solvent system may be selected from the group consisting of water, sulfoxides, preferably DMSO, ketones, preferably methyl ethylketone, methylisobutylketone and acetone, ethylene glycol ethers, preferably diethyleneglycol dimethyl ether (diglyme) or the reactant olefin or mixtures of two or more of the above solvents. Also so-called ionic liquids may be used. The latter refers to a class of inert ionic compounds with a low melting point, which may therefore be used as solvent. Examples thereof include e.g., 1-H-3-methyl imidazolium chloride, discussed in "Dehydration of fructose and sucrose into 5-hydroxymethylfurfural in the presence of 1-H-3-methyl imidazolium chloride acting both as solvent and catalyst", by Claude Moreau et al, Journal of Molecular Catalysis A: Chemical 253 (2006) 165-169.

The amount of solvent is preferably sufficient to dissolve the starting material and to limit undesired side-reactions.

The method of the current invention may be carried out in a batch process or in a continuous process, with or without recycle of (part of) the product stream to control the reaction temperature (recycle via a heat exchanger). For instance, the method of the invention can be performed in a continuous flow process. In such method, homogenous catalysts may be used and the residence time of the reactants in the flow process is between 0.1 second and 10 hours, preferably from 1 second to 1 hours, more preferably from 5 seconds to 20 minutes.

Alternatively, the continuous flow process may be a fixed bed continuous flow process or a reactive (catalytic) distillation process with a heterogeneous acid catalyst. To initiate or regenerate the heterogeneous acid catalyst or to improve performance, an inorganic or organic acid may be added to the feed of the fixed bed or reactive distillation continuous flow process. In a fixed bed process, the liquid hourly space velocity (LHSV) can be from 1 to 1000, preferably from 5 to 500, more preferably from 10 to 250 and most preferably from 25 to 100 $min^{-1}$.

The above process results in a stable HMF ether, which can then be used as such or be converted into a further derivative before being used as fuel and/or as fuel additive. The HMF ethers made according to the invention can also be used as or can be converted to compounds that can be used as solvent, as monomer in a polymerization (such as 2,5-furandicarboxylic acid or FDCA, as fine chemical or pharmaceutical intermediate, or in other applications. Oxidation of 5-(tertbutoxymethyl)furfural using an appropriate catalyst under appropriate conditions such as for example described for p-xylene with a $NHPI/Co(OAc)_2/MnOAc)_2$ catalyst system in Adv. Synth. Catal. 2001, 343, 220-225 or such as described for HMF with a Pt/C catalyst system at pH < 8 in EP 0 356 703 or or such as described for HMF with a Pt/C catalyst system at pH > 7 in FR 2 669 634, all with air as an oxidant, resulted in the formation of 2,5-furan dicarboxylic acid (FDCA).

The invention further describes the use of the HMF ethers prepared by the method of the current invention as fuel and/or as fuel additive. Of particular interest is the use of the ethers in diesel, biodiesel or "green diesel", given its (much) greater solubility therein than ethanol. Conventional additives and blending agents for diesel fuel may be present in the fuel compositions of this invention in addition to the above mentioned fuel components. For example, the fuels may contain conventional quantities of conventional additives such as cetane improvers, friction modifiers, detergents, antioxidants and heat stabilizers, for example. Especially preferred diesel fuel formulations comprise diesel fuel hydrocarbons and HMF ether as above described together with peroxidic or nitrate cetane improvers such as ditertiary butyl peroxide, amyl nitrate and ethyl hexyl nitrate for example.

The addition of this HMF ether to diesel fuel results in similar $NO_x$ emissions and slightly higher CO emissions; however, the use of sufficient amounts of cetane improvers can be utilized to improve the $NO_x$ and CO emissions well below the base reference fuel.

Examples are enclosed to illustrate the method of the current invention and the suitability of the products prepared there from as fuel. The examples are not meant to limit the scope of the invention.

**Example 1. tBMF formation.**

In a 7.5 ml batch reactor, 0.077 mmol HMF, dissolved in diglyme, was reacted with 0.3 mmol isobutene and 0.9 mg acid catalyst at a temperature 90,120 or 150 degrees Celsius for 1.5 or 6 hours. Two main furan peaks were observed in the UV spectra. Mass spectrometry identified these products as HMF and 5-(tertbutoxymethyl)furfural (tBMF). Selectivities and conversions for catalysts used in this example can be found in table below.

Conversion of substrate, selectivity and yield of furan derivatives were calculated according to the following formulae:

$$X = 100 * m_{r\ substrate} / m_{0\ substrate}$$

X conversion (%)
$m_{r\ substrate}$ amount of reacted substrate (mg)
$m_{0\ substrate}$ amount of substrate in feed (mg)

$$S_{compound} = 100 * n_{r\ substrate} / n_{0\ substrate}$$

$S_{compound}$ selectivity to compound (%)
$n_{r\ substrate}$ moles of substrate reacted
$n_{0\ substrate}$ moles of substrate in feed

$$Yield = 100 * n_{product} / n_{0\ substrate}$$

Yield yield (%)

$n_{product}$ moles of product formed

| Catalyst | T [°C] | Reaction Time [h] | Conversion [%] | Selectivity to tBMF[%] |
|---|---|---|---|---|
| Bentonite | 90 | 1.5 | 19.9 | 97.6 |
| H2SO4 | 90 | 1.5 | 33.0 | 68.6 |
| Sc(III) triflate | 90 | 1.5 | 47.6 | 51.3 |
| Sm(III) triflate | 90 | 1.5 | 49.3 | 48.8 |
| Y(III) triflate | 90 | 1.5 | 3.1 | 63.2 |
| Amberlyst36Dry | 90 | 1.5 | 71.8 | 23.8 |
| H2SO4 | 120 | 1.5 | 48.2 | 21.2 |
| Sc(III) triflate | 120 | 1.5 | 93.6 | 3.3 |
| Sm(III) triflate | 120 | 1.5 | 82.2 | 3.1 |
| Y(III) triflate | 120 | 1.5 | 59.5 | 3.7 |
| Amberlyst36Dry | 120 | 1.5 | 63.5 | 2.6 |
| H2SO4 | 150 | 6 | 100.0 | 0.5 |
| Sc(III) triflate | 150 | 6 | 81.6 | 0.2 |
| Sm(III) triflate | 150 | 6 | 59.5 | 0.6 |
| Y(III) triflate | 150 | 6 | 79.2 | 0.2 |
| Amberlyst36Dry | 150 | 6 | 100.0 | 0.2 |

## Analytical Method

The reaction products were quantified with the aid of HPLC-analysis with an internal standard (saccharine, Sigma Aldrich). An Agilent 1100 series chromatograph, equipped with UV and ELSD detectors, was used. Stationary phase was reverse phase C18 (Sunfire 3.5 $\mu$m, 4.6x100mm, Waters) column. A gradient elution at a constant flow 0.6 ml/min and temperature 40 °C was used according to the following scheme.

| Time | H2O(vol%) | MeOH (vol%) | MeCN (vol%) | Flow (ml/min) | T (C) |
|---|---|---|---|---|---|
| Initial | 95 | 0 | 5 | 1 | 40 |
| 1 | 89 | 3 | 8 | 1 | 40 |
| 8 | 25 | 3 | 72 | 1 | 40 |

The product was characterized with 1 H NMR and LC-MS (CI) (See pictures below). Molecular mass of tBMF is 182.2 g/mol. See fig. 1.

**Example 2. diesel fuel applications** (Reference)

Fuel solubility

Fuel solubility is a primary concern for diesel fuel applications. Not all highly polar oxygenates have good solubility in the current commercial diesel fuels. Results show that in the 5 vol%, in the 25 vol% and in the 40 vol% blends of tBMF with commercial diesel, both liquid blend components are completely miscible. In a comparative set of experiments it was shown that ethoxymethylfurfural (EMF) is completely miscible in a 5 vol% blend with commercial diesel, but that phase separation occurs with the 25 vol% and with the 40 vol% blends of EMF and diesel.

Cetane number

[0036] Oxygenated fuel additives may reduce the natural cetane number of the base diesel fuel. A 0.1 vol% blend of tBMF with additive free diesel fuel was prepared at an outside laboratory for cetane determination according to an ASTM D 6890 certified method. While the reference additive-free diesel showed an average cetane number of 52.5, surprisingly, the 0.1 vol% tBMF blend showed an increase with 0.5 to an average cetane number of 53.0.

Oxidation stability

Likewise, oxygenated fuel additives, certainly when containing an aldehyde functional group, often reduce the oxidation stability of the base diesel fuel. A 0.1 vol% blend of tBMF with additive free diesel fuel was prepared at an outside laboratory for oxidation stability determination according to NF en ISO 12205 certified methods. Surprisingly, both the reference additive-free diesel and the 0.1 vol% tBMF blend showed the same oxidation stability, indicating that the oxygenated tMBF added to an additive free diesel base fuel does not decrease the oxidation stability of the blend relative to the pure base diesel.

**Example 3. Emission engine testing** (Reference)

In a D9B diesel engine of a citroen Berlingo test car, comparative testing is performed with normal commercial diesel as a fuel and the same commercial diesel to which 25 vol. % 5-(t-butoxymethyl)furfural (tBMF) was added, respectively. tBMF is added as a liquid and does not yield any mixing or flocculation problems up to a 40 vol% blend ratio. The engine is run stationary with regular diesel initially, after which the fuel supply is switched to the 40 vol% tBMF-diesel blend.

During stationary operation with the commercial diesel fuel and with the 25 vol% tBMF blend, the following measurements were made: total particulate matter, volume, $O_2$, CO, $CO_2$, $NO_x$ (NO + $NO_2$) and total hydrocarbons.

Total particulate matter was sampled according to NEN-EN 13284-1

Particle size distribution was sampled according to VDI 2066-5

Volume was measured according to ISO 10780

Gases were sampled according to ISO 10396

$O_2$, CO and $CO_2$ were analysed according to NEN-ISO 12039

$NO_x$ (NO + $NO_2$) was analysed according to NEN-ISO 10849

Total hydrocarbons were analysed according to NEN-EN 13526.

Table 1. gas analysis results of 100% commercial diesel fuel.

| Experiment | Component | Average Concentration | Emission |
|---|---|---|---|
| 1 | CO | 191 mg/Nm$^3$ | 13 g/h |
| | $CO_2$ | 2.4 % v/v | - |
| | $O_2$ | 17.8% v/v | - |
| | $NO_x$ | 323 mg/Nm$^3$ | 21 g/h |

Table 2, particulate matter results of 100% commercial diesel fuel.

| Experi ment | Volume | | Total particulate matter | | Particle size |
|---|---|---|---|---|---|
| | Actual [m3/h] | Normal [Nm3/h] | Concentration [mg/Nm3] | Emission [g/h] | PSD <10 $\mu$m [%] |
| 1 | 80 | 60 | 6.1 | | 98,5 |

Table 3. gas analysis results of blend of commercial diesel with 25 vol% tBMF.

| Experiment | Component | Average Concentration | Emission |
|---|---|---|---|
| 2 | CO | 243 mg/Nm$^3$ | 16 g/h |
| | $CO_2$ | 2.5 % v/v | - |
| | $O_2$ | 17.7 % v/v | - |
| | $NO_x$ | 333 mg/Nm$^3$ | 22 g/h |

Table 4, particulate matter results of blend of commercial diesel with 25 vol% tBMF.

| Experiment | Volume | | Total particulate matter | | Particle |
|---|---|---|---|---|---|
| | Actual [m3/h] | Normal [Nm3/h] | Concentration [mg/Nm3] | Emission [g/h] | PSD <10 $\mu$m [%] |
| 3b (**) | 80 | 60 | 5.1 | | 100 |

Example 4

In a typical experiment, 0.27 mmol of HMF, 0.8 ml of diglyme and 0.1 ml 2-methyl-1-pentene (MP) were added in a reactor, coated inside with Teflon. The mixture reacted under nitrogen (12.5 bar) in the presence of a acid catalyst (6.5 mg) for 4 h at 90 °C. The main peaks observed in the UV spectrum were identified as HMF and 5-(1,1-dimethylbutoxymethyl)furfural (MPMF). In this experiment, the selectivity was calculated slightly different, based on the formula:

$$\textbf{Selectivity} = 100 * n_t \text{ (product)} / [n_0 \text{ (substrate)} - n_t \text{ (substrate)}]$$

Where:

$n_0$- the initial number of moles

$n_t$- the number the moles of a compound at time "t".

| Catalyst | Conv. (%) | MPMF select. (%) |
|---|---|---|
| $H_2SO_4$ | 47.1 | 45.9 |
| Sc(III) triflate | 60.6 | 36.8 |
| Bentonite | 22.9 | 52.1 |
| Sm(III) triflate | 44.7 | 41.9 |
| Y(III) triflate | 31.3 | 32.2 |
| Al(III) triflate | 73.5 | 32.1 |
| Montmorillonite K 5 | 24.0 | 42.7 |

References

• DUMESIC, James A, et al. "Phase modifiers promote efficient production of Hydroxymethylfurfural from fructose" . Science. 30 June 2006, vol.312, no.5782, p.1933-1937.
• WO 2006/063220
• Chapter 15 of Advanced Organic Chemistry, by Jerry March, and in particular under reaction 5-4. (3rd ed., © 1985 by John Wiley & Sons, pp. 684-685).
• DE FREITAS, Filho J.R. et al, "Synthesis of new 2,3-unsaturated o-glycosides through ferrier rearrangement", in Journal of Carbohydrate Chemistry, 2001, vol. 20, pages 561-568.
• LEWKOWSKI, Jaroslaw. Synthesis, chemistry and applications of 5-hydroxymethylfurfural and its derivatives. Arkivoc. 2001, p.17-54.
• MOREAU, Claude, et al. "Dehydration of fructose and sucrose into 5-hydroxymethylfurfural in the presence of 1-H-3-methyl imidazolium chloride acting both as solvent and catalyst", Journal of Molecular Catalysis A: Chemical 253 (2006) p. 165-169.

• EP 0641 854
• UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05G015085))
• Adv. Synth. Catal. 2001, 343, 220-225

- EP 0 356 703
- FR 2 669 634

**Claims**

1. Method for the manufacture of an ether of 5-hydroxymethylfurfural through hydro-alkoxy-addition by reacting 5-hydroxymethylfurfural with an olefin in the presence of an acid or acidic catalyst, wherein the olefin is represented by the general formulae

$$HRC=CR'R''\ H2C=CR'R''$$

wherein each R, R' and R" independently represents an alkyl, aralkyl and alkenyl group which may be linear, branched or cyclic with up to 7 carbon atoms, or R and R' jointly represent an alkenyl group with up to 7 carbon atoms.

2. Method according to claim 1, wherein the olefin is selected from one or more of the group comprising isobutene, pinene and limonene.

3. Method according to claim 2, wherein the olefin is isobutene.

4. Method according to any one of claims claim 1 to 3, wherein the acid catalyst is selected from the group consisting of homogeneous or heterogeneous acids selected from solid organic acids, inorganic acids, salts, Lewis acids, ion exchange resins, zeolites or mixtures and/or combinations thereof.

5. Method according to any one of the claims 1 to 4, wherein the reaction is performed at a temperature from 50 to 200 degrees Celsius, preferably from 50 to 150, more preferably from 75 to 125 degrees Celsius.

6. Method according to any one of the claims 1 to 5, wherein the HMF used as a starting material is obtained from biomass.

7. Method according to claims 6, wherein the HMF used as a starting material is obtained from the sucrose, glucose, fructose or mixtures thereof.

8. Method according to any one of the claims 1 to 7, performed in the presence of a solvent, wherein the solvent or solvents are selected form the group of non protic polar solvents consisting of sulfoxides, preferably DMSO, ketones, preferably methyl ethylketone, methylisobutylketone and/or acetone, esters, ethers, preferably ethylene glycol ethers, more preferably diethyleneglycol dimethyl ether (diglyme) or the reactant olefin and mixtures thereof.

9. Method according to any one of the claims 1 to 8, wherein the method is performed in a continuous flow process.

10. Method according to claim 9, wherein the residence time in the flow process is between 0.1 second and 10 hours, preferably from 1 second to 1 hours, more preferably from 5 seconds to 20 minutes

11. Method according to claim 9, wherein, the continuous flow process is a fixed bed continuous flow process.

12. Method according to claim 9, wherein the fixed bed comprises a heterogeneous acid catalyst.

13. Method according to claim 9, wherein the continuous flow process is a reactive distillation or a catalytic distillation process.

14. Method according to claim 9, wherein in addition to a heterogeneous acid catalyst, an inorganic or organic acid catalyst is added to the feed of the fixed bed or catalytic distillation continuous flow process.

15. Method according to claim 9, wherein the liquid hourly space velocity ("LHSV") is from 1 to 1000, preferably from 5 to 500, more preferably from 10 to 250 and most preferably from 25 to 100.

**EP 2 197 865 B1**

**Patentansprüche**

1. Verfahren zur Herstellung eines 5-Hydroxymethylfurfuralethers mittels Hydroalkoxyaddition durch Umsetzen von 5-Hydroxymethylfurfural mit einem Olefin in Gegenwart einer Säure oder eines sauren Katalysators, wobei das Olefin durch die allgemeinen Formeln

   HRC=CR'R"

   H2C=CR'R"

   dargestellt ist, wobei jedes R, R' und R" unabhängig voneinander eine Alkyl-Aralkyl- und Alkenylgruppe darstellt, die linear, verzweigt oder zyklisch mit bis zu 7 Kohlenstoffatomen sein kann, oder wobei R und R' gemeinsam eine Alkenylgruppe mit bis zu 7 Kohlenstoffatomen darstellen.

2. Verfahren nach Anspruch 1, wobei das Olefin aus einer oder mehreren Verbindungen aus der Gruppe, umfassend Isobuten, Pinen und Limonen ausgewählt ist.

3. Verfahren nach Anspruch 2, wobei das Olefin Isobuten ist.

4. Verfahren nach einem der Ansprüche 1 bis 3, wobei der saure Katalysator ausgewählt ist aus der Gruppe bestehend aus homogenen oder heterogenen Säuren, die aus festen organischen Säuren, anorganischen Säuren, Salzen, LewisSäuren, Ionenaustauscherharzen, Zeolithen oder Mischungen und/oder Kombinationen davon ausgewählt sind.

5. Verfahren nach einem der Ansprüche 1 bis 4, wobei die Reaktion bei einer Temperatur von 50 bis 200 °C, vorzugsweise von 50 bis 150, bevorzugter von 75 bis 125 °C durchgeführt wird.

6. Verfahren nach einem der Ansprüche 1 bis 5, wobei das als Ausgangsmaterial verwendete HMF aus Biomasse erhalten wird.

7. Verfahren nach Anspruch 6, wobei das als Ausgangsmaterial verwendete HMF aus Sucrose, Glucose, Fructose oder Mischungen davon erhalten wird.

8. Verfahren nach einem der Ansprüche 1 bis 7, durchgeführt in Gegenwart eines Lösungsmittels, wobei das Lösungsmittel oder die Lösungsmittel ausgewählt sind aus der Gruppe der aprotischen polaren Lösungsmitteln, bestehend aus Sulfoxiden, vorzugsweise DMSO, Ketonen, vorzugsweise Methylethylketon, Methylisobutylketon und/oder Aceton, Estern, Ethern, vorzugsweise Ethylenglycolethern, bevorzugter Diethylenglycoldimethylether (Diglyme) oder dem Olefinreaktanden und Mischungen davon.

9. Verfahren nach einem der Ansprüche 1 bis 8, wobei das Verfahren als kontinuierliches Strömungsverfahren durchgeführt wird.

10. Verfahren nach Anspruch 9, wobei die Verweilzeit in dem Strömungsverfahren zwischen 0,1 Sekunden und 10 Stunden, vorzugsweise 1 Sekunde bis 1 Stunde, bevorzugter 5 Sekunden bis 20 Minuten beträgt.

11. Verfahren nach Anspruch 9, wobei das kontinuierliche Strömungsverfahren ein kontinuierliches Festbettverfahren ist.

12. Verfahren nach Anspruch 9, wobei das Festbett einen heterogenen sauren Katalysator umfasst.

13. Verfahren nach Anspruch 9, wobei das kontinuierliche Strömungsverfahren ein reaktives Destillations- oder ein katalytisches Destillationsverfahren ist.

14. Verfahren nach Anspruch 9, wobei zusätzlich zu einem heterogenen sauren Katalysator ein anorganischer oder organischer saurer Katalysator zur Einspeisung des Festbetts oder des kontinuierlichen katalytischen Destillationsverfahrens zugegeben wird.

15. Verfahren nach Anspruch 9, wobei die flüssigkeitsbezogene Raumgeschwindigkeit pro Stunde ("liquid hourly space velocity", "LHSV") 1 bis 1000, vorzugsweise 5 bis 500, bevorzugter 10 bis 250 und besonders bevorzugt 25 bis 100 beträgt.

**Revendications**

1. Procédé de fabrication d'un éther de 5-hydroxyméthylfurfural au moyen d'une hydroalcoxy-addition, par réaction de 5-hydroxyméthylfurfural avec une oléfine en présence d'un acide ou d'un catalyseur acide, dans lequel l'oléfine est représentée par les formules générales

HRC=CR'R" H2C=CR'R"

dans lesquelles chaque R, R' et R" représente indépendamment un groupe alkyle, aralkyle ou alcényle qui peut être linéaire, ramifié ou cyclique en ayant jusqu'à 7 atomes de carbone, ou bien R et R' représentent ensemble un groupe alcényle ayant jusqu'à 7 atomes de carbone.

2. Procédé selon la revendication 1, dans lequel l'oléfine est choisie parmi un ou plusieurs membres du groupe comprenant l'isobutène, le pinène et le limonène.

3. Procédé selon la revendication 2, dans lequel l'oléfine est l'isobutène.

4. Procédé selon l'une quelconque des revendications 1 à 3, dans lequel le catalyseur acide est choisi dans le groupe constitué par les acides homogènes et hétérogènes choisis parmi les acides organiques solides, les acides inorganiques, les sels, les acides de Lewis, les résines échangeuses d'ions, les zéolites et leurs mélanges et/ou combinaisons.

5. Procédé selon l'une quelconque des revendications 1 à 4, dans lequel la réaction est effectuée à une température de 50 à 200°C, de préférence de 50 à 150, mieux encore de 75 à 125°C.

6. Procédé selon l'une quelconque des revendications 1 à 5, dans lequel le HMF utilisé en tant que matériau de départ est obtenu à partir d'une biomasse.

7. Procédé selon la revendication 6, dans lequel le HMF utilisé en tant que matériau de départ est obtenu à partir de saccharose, glucose, fructose ou leurs mélanges.

8. Procédé selon l'une quelconque des revendications 1 à 7, mis en oeuvre en présence d'un solvant, dans lequel le ou les solvants sont choisis dans le groupe constitué par les solvants polaires non protiques constitués par les sulfoxydes, de préférence le DMSO, les cétones, de préférence la méthyléthylcétone, la méthylisobutylcétone et/ou l'acétone, les esters, les éthers, de préférence les éthers d'éthylèneglycol, mieux encore l'éther diméthylique de diéthylèneglycol (diglyme), et l'oléfine servant de réactif, ainsi que leurs mélanges.

9. Procédé selon l'une quelconque des revendications 1 à 8, lequel procédé est mis en oeuvre selon un procédé à écoulement en continu.

10. Procédé selon la revendication 9, dans lequel le temps de séjour dans le procédé à écoulement est compris entre 0,1 seconde et 10 heures, de préférence entre 1 seconde et 1 heure, mieux encore entre 5 secondes et 20 minutes.

11. Procédé selon la revendication 9, dans lequel le procédé à écoulement en continu est un procédé à écoulement en continu en lit fixe.

12. Procédé selon la revendication 9, dans lequel le lit fixe comprend un catalyseur acide hétérogène.

13. Procédé selon la revendication 9, dans lequel le procédé à écoulement en continu est un procédé de distillation réactive ou de distillation catalytique.

14. Procédé selon la revendication 9, dans lequel, en plus d'un catalyseur acide hétérogène, un catalyseur acide inorganique ou organique est ajouté à la charge du lit fixe ou du procédé à écoulement en continu par distillation catalytique.

**15.** Procédé selon la revendication 9, dans lequel la vitesse spatiale horaire de liquide ("VSHL") est de 1 à 1000, de préférence de 5 à 500, mieux encore de 10 à 250 et tout spécialement de 25 à 100.

*MSD1 SPC, time=7.754 of AMAZONE\P0817005.D    API-ES, Pos, Scan, Frag: 70

Max: 45104

Fig. 2

EP 2 197 865 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0641854 A
- WO 2006063220 A
- EP 0356703 A
- FR 2669634

**Non-patent literature cited in the description**

- **DUMESIC, James A et al.** Phase modifiers promote efficient production of Hydroxymethylfurfural from fructose. *Science,* 30 June 2006, vol. 312 (5782), 1933-1937
- **Jerry March.** Advanced Organic Chemistry. John Wiley & Sons, 1985, 684-685
- **Filho J.R. DE FREITAS et al.** Synthesis of new 2,3-unsaturated o-glycosides through ferrier rearrangement. *Journal of Carbohydrate Chemistry,* 2001, vol. 20, 561-568
- **Mr. Lewkowski.** Synthesis, chemistry and applications of 5-hydroxymethylfurfural and its derivatives. *Arkivoc.,* 2001, 17-54
- **Claude Moreau et al.** Dehydration of fructose and sucrose into 5-hydroxymethylfurfural in the presence of 1-H-3-methyl imidazolium chloride acting both as solvent and catalyst. *Journal of Molecular Catalysis A: Chemical,* 2006, vol. 253, 165-169
- *Adv. Synth. Catal.,* 2001, vol. 343, 220-225
- **DE FREITAS, Filho J.R. et al.** Synthesis of new 2,3-unsaturated o-glycosides through ferrier rearrangement. *Journal of Carbohydrate Chemistry,* 2001, vol. 20, 561-568
- **LEWKOWSKI, Jaroslaw.** Synthesis, chemistry and applications of 5-hydroxymethylfurfural and its derivatives. *Arkivoc.,* 2001, 17-54
- **MOREAU, Claude et al.** Dehydration of fructose and sucrose into 5-hydroxymethylfurfural in the presence of 1-H-3-methyl imidazolium chloride acting both as solvent and catalyst. *Journal of Molecular Catalysis A: Chemical,* 2006, vol. 253, 165-169